# FASCICULE DE BREVET EUROPEEN

(11) **EP 2 209 005 B1**
(45) Date de publication et mention de la délivrance du brevet: **28.02.2018**
(21) Numéro de dépôt: 10150605.3
(22) Date de dépôt: 13.01.2010
(51) Int. Cl.: A61K 8/64, A61Q 19/08, G01N 33/68

(54) **Utilisation de formes complexes de la protéine de type calmodulin-like skin protein CLSP**
Einsatz der komplexen Formen des Proteins vom Typ CLSP (calmodulin-like skin protein)
Use of complex forms of the calmodulin-like skin protein CLSP

(30) Priorité: 13.01.2009 FR 0950162; 27.01.2009 US 202077 P
(43) Date de publication de la demande: 21.07.2010
(60) Demande divisionnaire: 12176580.4
(73) Titulaire: L'Oréal, 75008 Paris (FR)
(72) Inventeur: Donovan, Mark, 95810 Berville (FR); Bernard, Dominique, 92170 Vanves (FR)
(74) Mandataire: Nony

(56) Documents cités:
- FR-A1- 2 796 646
- URSCHITZ JOHANN; ET AL: "A serial analysis of gene expression in sun-damaged human skin" JOURNAL OF INVESTIGATIVE DERMATOLOGY, NATURE PUBLISHING GROUP, GB, vol. 119, no. 1, 1 juillet 2002 (2002-07-01), pages 3-13, XP002487724 ISSN: 0022-202X
- BAUMANN L; WEINKLE S: "Improving elasticity: The science of aging skin" COSMETIC DERMATOLOGY 200703 US, vol. 20, no. 3, mars 2007 (2007-03), pages 168-172, XP009122793 ISSN: 1041-3766
- MÉHUL BRUNO; BERNARD DOMINIQUE; BROUARD MICHEL; DELATTRE CAROLINE; SCHMIDT RAINER: "Influence of calcium on the proteolytic degradation of the calmodulin-like skin protein (calmodulin-like protein 5) in psoriatic epidermis." EXPERIMENTAL DERMATOLOGY JUN 2006, vol. 15, no. 6, juin 2006 (2006-06), pages 469-477, XP002546044 ISSN: 0906-6705

## Description

La présente invention a pour objet principal l'utilisation de formes complexes de la protéine calmodulin-like skin protein (CLSP), de fragments peptidiques de cette protéine ou d'analogues de celle-ci, à titre de marqueur pour l'évaluation d'un état de vieillissement chronologique d'un épithélium, et notamment de l'épiderme.

Les épithéliums sont des tissus dont les cellules sont jointives et solidaires les unes des autres et reposent sur une membrane basale. Ils forment un revêtement soit externe, par exemple en surface de la peau, ou l'épiderme, soit interne, en surface d'une muqueuse. Ils peuvent également former des glandes.

Plus précisément, ces épithéliums sont des structures dont l'homéostasie résulte de la mise en oeuvre d'un ensemble, finement régulé, de signaux intracellulaires et extracellulaires agissant à toutes les étapes de la prolifération, de la migration, de la différenciation cellulaire, ainsi que de la synthèse des différents composants de la matrice extracellulaire. Ces signaux peuvent, notamment, résulter de l'action de facteurs produits par des kératinocytes.

Le maintien des bonnes fonctions physiologiques d'un épithélium implique notamment la différenciation épithéliale terminale et/ou la synthèse de protéoglycannes.

En ce qui concerne plus particulièrement l'épiderme, il s'agit d'un épithélium, conventionnellement divisé en une couche basale de kératinocytes contenant, notamment, des cellules souches cutanées et constituant la couche germinative de l'épiderme, une couche dite épineuse constituée de plusieurs couches de cellules polyédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée (ou *stratum corneum*) constituée de kératinocytes au stade terminal de leur différenciation appelés cornéocytes.

Le *stratum corneum,* la partie la plus externe de la peau qui assure la fonction barrière entre l'organisme et l'environnement, et la tige pilaire, partie émergente du follicule pileux qui constitue la chevelure, représentent tous deux l'aboutissement du processus de différenciation des kératinocytes. La différenciation épidermique suit un processus de maturation dans lequel des kératinocytes de la couche basale se différencient et migrent pour aboutir à la formation des cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme.

De nombreux troubles ou pathologies cutanées peuvent résulter d'un dysfonctionnement de l'homéostasie de l'épiderme.

Ainsi, dans le cas de la peau âgée, ce dysfonctionnement se manifeste généralement par l'apparition de rides (micro-relief et rides profondes), une perte de l'élasticité, un toucher rugueux et une sécheresse. Sur le plan histologique, un aplatissement de la jonction dermo-épidermique et une diminution d'épaisseur du derme et de l'épiderme sont observés. Le contenu en collagène et en glycosaminoglycannes diminue. La fonction barrière de la peau est altérée. L'ensemble de ces phénomènes est accru par l'exposition solaire chronique.

De même, ce dysfonctionnement peut être exacerbé chez les femmes, lors de la ménopause.

La présente invention résulte plus particulièrement de la caractérisation par les inventeurs au niveau du *stratum corneum,* de formes complexes de la protéine CLSP, dont l'expression s'avère être modulée de manière différente au cours du vieillissement chronobiologique d'un épithélium.

La CLSP est une protéine de liaison au calcium appartenant à la famille des calmodulines dont la forme précurseur comprend 146 acides aminés. Elle présente un poids moléculaire d'environ 16 kDa et une homologie de séquences d'acides aminés d'environ 52 % avec la calmoduline.

La structure de la CLSP comprend 4 sites de liaison potentiels au calcium de type « EF-hand », des sites potentiels de N-glycosylation, de multiples sites potentiels de phosphorylation et un site de myristylation.

La liaison du calcium à la CLSP induit un changement de sa conformation qui entraîne son activation, en permettant notamment son interaction avec différentes protéines cibles.

Il a été montré une augmentation de l'ARNm codant pour la CLSP au cours du vieillissement photo-induit (Urschitz, J. et al., J. Invest. Dermatol., 2002, 119 :3-13) c'est-à-dire du vieillissement résultant d'une exposition répétée de la peau aux rayons du soleil, un processus distinct de celui du vieillissement chronologique, c'est-à-dire du vieillissement physiologique lié à l'écoulement du temps.

Pour sa part, le document US 2004/0142335 observe par le biais d'une analyse transcriptomique une augmentation de l'expression de l'ARNm codant pour la protéine CLSP dans une peau d'un individu âgé comparativement à celle d'un individu jeune.

Ainsi, la CLSP pourrait notamment être impliquée dans la différenciation terminale des kératinocytes (B. Méhul et al., J. Biol. Chem., 2000, Apr. 28 ; 275(17) :12841-7), (EP 1 204 744).

Par ailleurs, le document FR2796646 suggère l'existence de formes complexes de la CLSP et propose l'utilisation de la protéine CLSP pour traiter le vieillissement cutané. Il est toutefois totalement silencieux quant au vieillissement chronologique cutané et quant à une quelconque relation entre formes complexes de la CLSP et vieillissement cutané.

Plus récemment, les inventeurs ont, de manière inattendue, constaté une diminution de l'expression de formes particulières de la CLSP dans le *stratum corneum* de l'épiderme humain âgé. Manifestement, les formes caractérisées par les inventeurs correspondent à des stades de maturation de la protéine distincts de ceux des formes de CLSP jusqu'ici considérées et notamment évoquées ci-dessus.

Comme il ressort de ce qui suit, les formes complexes ont été caractérisées par les inventeurs grâce à la mise en oeuvre d'une technique de dosage de type ELISA spécifique, notamment décrite dans l'exemple 1, ci-après.

Ainsi, selon un de ses aspects, la présente invention concerne l'utilisation d'au moins une forme complexe de la Calmoduline-like skin protein ou CLSP représentée par un polypeptide de séquence d'acides aminés codée par une séquence d'acides nucléiques représentée par SEQ ID NO 1, ou un analogue présentant une homologie de séquence d'au moins 85 % avec ledit polypeptide, ainsi qu'une activité biologique de même nature, ou un fragment de protéolyse de la CLSP de séquence peptidique choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 et SEQ ID NO 25, conjugué avec soit une protéine distincte de la CLSP, soit une autre protéine CLSP, soit un fragment de protéolyse de ladite autre protéine CLSP de séquence peptidique choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 et SEQ ID NO 25, à titre d'outil de caractérisation *in vitro* ou *ex vivo* d'un état de vieillissement chronologique d'un épithélium, et notamment d'un épiderme, une diminution en forme(s) complexe(s) étant indicative d'un état de vieillissement chronologique.

Par « vieillissement chronologique », au sens de l'invention, on entend écarter de la portée de l'invention les phénomènes cutanés consécutifs d'une exposition au soleil ou aux U.V., à savoir le vieillissement cutané photo-induit.

Au sens de la présente invention, le terme « forme complexe » entend couvrir tout conjugué de la protéine CLSP ou dérivé de celle-ci, avec soit une autre protéine distincte de la CLSP ou un fragment de celle-ci, soit une autre protéine CLSP ou un de ses fragments.

Au sens de la présente invention, le terme « dérivé de la protéine CLSP » désigne un fragment de celle-ci ou un analogue tel que défini ci-après.

Plus précisément, une forme complexe peut dériver de l'association de la protéine CLSP entière ou de l'un de ses fragments avec soit elle-même, soit l'un de ses fragments, ou encore avec une protéine cible, voire avec une structure protéique de type enveloppe cornifiée protéolysée dans le *stratum corneum.*

Ainsi, selon une première variante, les formes complexes considérées selon l'invention peuvent être des formes dimères de la protéine CLSP, ou encore le conjugué de deux fragments peptidiques, identiques ou différents, de cette protéine.

Selon une autre variante, ces formes complexes peuvent être le produit résultant de l'association de la protéine CLSP ou l'un de ses fragments, avec une protéine cible annexe. A titre d'illustration de ces protéines cibles susceptibles d'interagir avec la CLSP et/ou l'un de ses fragments, peuvent notamment être cités le polypeptide 14.3.3 Beta, le 14.3.3 Sigma, les annexines II et V, les calréticulines, l'ERp72, la Nucléoline, la Transglutaminase 3, la MAGED1 (Melanoma antigen gene D1), le PPP4C (Protein phosphatase 4) et l'USF2 (Upstream transcription factor 2). Il peut également s'agir de motifs peptidiques tels que le motif YWHAQ (14.3.3 Tau).

Contre toute attente, les inventeurs ont constaté que la quantification de ces formes complexes dans le *stratum corneum* constitue un moyen efficace et fiable pour qualifier l'état de l'épiderme correspondant. Comme précisé ci-dessus, il est constaté une diminution de l'expression de ces formes complexes au cours du vieillissement chronologique.

A ce titre, une diminution en forme(s) complexe(s) conforme(s) à l'invention est indicative d'un état de vieillissement chronologique d'un épithélium.

La caractérisation de ces formes complexes s'avère donc particulièrement utile pour établir un diagnostic de l'état d'un épiderme.

Au regard de ce diagnostic, il pourra être avantageusement proposé au sujet un traitement cosmétique tout particulièrement ajusté à son profil. Pour des raisons évidentes, ce type de personnalisation en termes de traitement cosmétique est particulièrement recherché et apprécié de la part des utilisateurs. La présente invention permet avantageusement de répondre à cette demande.

Selon un autre de ses aspects, la présente invention concerne un procédé, in vitro ou ex vivo, de caractérisation d'un état d'un épithélium comprenant au moins les étapes consistant à :
a) déterminer dans un échantillon de surface dudit épithélium, une teneur en au moins une forme complexe de l'invention, et
b) comparer ladite teneur déterminée à l'étape a) à une valeur de référence,
une diminution en forme(s) complexe(s) étant indicative d'un état de vieillissement chronologique.

Le présent texte décrit également un procédé, notamment cosmétique, non invasif, pour caractériser, notamment *in vitro* ou *ex vivo,* l'état chronologique d'un épiderme, comprenant au moins la caractérisation qualitative ou quantitative au niveau du *stratum corneum* dudit épiderme de l'expression d'au moins une forme complexe de la CLSP selon l'invention.

Selon une variante de réalisation, la donnée ou valeur obtenue via un tel procédé peut être appréciée comparativement à une donnée ou valeur de référence, obtenue par exemple à partir d'au moins un épithélium, notamment un épiderme, distinct de celui faisant l'objet de la caractérisation, et dont l'état est connu.

Selon un de ses aspects, le présent texte décrit un procédé pour caractériser l'état chronologique d'un épiderme tel que cité ci-dessus, dans lequel une diminution en formes complexes conformes à l'invention est indicative d'un état de vieillissement chronologique.

Le présent texte décrit encore un procédé, notamment cosmétique, non invasif, pour caractériser, notamment *in vitro* ou *ex vivo,* l'efficacité d'un traitement, cosmétique ou thérapeutique, visant à prévenir et/ou traiter les signes de vieillissement cutané liés au vieillissement chronologique chez un individu, tels que les rides et ridules, comprenant au moins la caractérisation qualitative ou quantitative d'au moins une forme complexe de la CLSP selon l'invention.

Par « signes de vieillissement cutané », on entend toutes les modifications de l'aspect extérieur de la peau dues au vieillissement chronologique, comme par exemple les rides et ridules, la peau flétrie, le manque d'élasticité et/ou de tonus de la peau, l'amincissement du derme et/ou la dégradation des fibres de collagène ce qui entraîne l'apparence de peau molle et ridée.

Les signes de vieillissement cutané considérés dans l'invention sont ceux associés au ou résultant du vieillissement cutané chronologique.

En particulier, les signes de vieillissement cutané considérés dans l'invention sont distincts de ceux associés au ou résultant du vieillissement cutané photo-induit.

Par vieillissement photo-induit, on entend notamment désigner les dommages oxydatifs liés à l'exposition de l'épiderme aux UV, et notamment aux UVA, entraînant la synthèse de radicaux libres, la glycation des protéines ou l'activation excessive de certains récepteurs de surface des cellules, et sur le plan tissulaire, l'épaississement des fibres d'élastine.

La présente invention vise donc à prévenir et/ou traiter les manifestations cutanées liées au vieillissement chronologique, et plus particulièrement non liées à une exposition aux UV.

La présente invention s'adresse plus particulièrement aux manifestations du vieillissement cutané chronologique résultant de l'usure et de la sénescence cellulaire.

Notamment, le vieillissement chronologique se manifeste, sur le plan des cellules de l'épiderme, par une réduction des télomères, une diminution de la stimulation cellulaire en raison d'un déficit en différentes hormones, telles que la DHEA, ou en facteurs de croissance, ou en raison d'une diminution du nombre de récepteurs cellulaires, et, sur le plan tissulaire, notamment par la disparition des fibres d'élastines.

De tels phénomènes ne sont pas associés à une exposition aux UV, et donc au photo-vieillissement de la peau.

Les déficiences cellulaires et tissulaires consécutives au vieillissement chronologique de l'épiderme peuvent se manifester, notamment, par une xérose, un relâchement de l'épiderme, des rides et/ou ridules, un affaissement cutané, une kératose séborrhéique ou un angiome cerise.

Plus précisément, le procédé tel que défini ci-dessus peut comprendre au moins les étapes consistant à :
i. Disposer d'au moins un premier échantillon de surface cutané représentatif dudit individu,
ii. Quantifier au niveau dudit échantillon, notamment via une technique de dosage ELISA, et en particulier celle décrite en exemple 1 ci-après, au moins une forme complexe selon l'invention,
iii. Renouveler les étapes i. et ii. sur un deuxième échantillon de surface cutané représentatif dudit individu, et
iv. Comparer les résultats obtenus à l'issu des étapes ii. et iii., notamment afin d'en déduire une information relative à au moins un effet du traitement,
lesdits premier et second échantillons de surface cutané correspondant à des stades de traitement différents.

La valeur ou donnée de référence à l'étape ii. peut être une donnée obtenue à partir de l'épithélium, notamment de l'épiderme, représentatif de l'individu faisant l'objet du traitement, antérieurement à l'administration dudit traitement ou dans un délai chronologique plus court au regard de la date de début de traitement.

Selon un mode de réalisation préférée, le premier échantillon est représentatif d'un état pré-traitement et le second est représentatif d'un état en cours de traitement ou post-traitement.

Le présent texte décrit l'un des procédés précédents, dans lequel une augmentation en forme(s) complexe(s) est indicative d'une efficacité dudit traitement des signes de vieillissement cutané.

Selon une variante de réalisation, l'un des procédés précédents peut comprendre à l'issue de l'étape de caractérisation des formes complexes de la CLSP, en outre au moins une étape complémentaire visant à administrer audit sujet une composition de soin, notamment cosmétique, établie ou sélectionnée au regard de l'information obtenue sur lesdites formes complexes. Cette étape complémentaire peut être consécutive à l'étape de caractérisation.

Selon un mode de réalisation, cette composition peut être aussi sélectionnée parmi une gamme de compositions, chacune de celles-ci étant plus particulièrement adaptées à un type d'information susceptible d'être obtenue selon le procédé de l'invention.

Ainsi, la présente invention a pour intérêt de proposer une méthode simple et rapide d'une part, pour établir la caractérisation de l'état physiologique d'un épithélium, en particulier de l'épiderme de la peau et, d'autre part, pour ajuster en conséquence un traitement adéquat audit épithélium ou épiderme de la peau.

Les procédés de l'invention peuvent être effectués *in vitro, ex vivo*

Comme il ressort de la description qui suit, les procédés selon l'invention sont particulièrement avantageux dans la mesure où leur mise en oeuvre ne requiert pas d'opération invasive.

En effet, la localisation par les inventeurs de ces nouveaux biomarqueurs du vieillissement dans le *stratum corneum* rend possible une caractérisation quantitative ou qualitative de l'expression de ceux-ci par simple prélèvement topique.

De manière avantageuse, ce procédé peut être réalisé sur un échantillon de *stratum corneum* du sujet considéré, simplement prélevé par stripping. La méthode de prélèvement peut par exemple être une technique de type stripping consistant à appliquer sur l'épithélium considéré, tel qu'un épiderme, une portion de ruban adhésif. En décollant ce ruban adhésif, une fraction de l'épithélium, par exemple une fraction épidermique, est prélevée. Après extraction protéique, celle-ci est ensuite analysée par un test ELISA tel que considéré dans la présente invention.

Par ailleurs, le présent texte décrit également l'utilisation d'au moins une forme complexe conforme à l'invention, à titre d'outil de criblage de composés biologiques ou chimiques susceptibles de moduler, et en particulier de favoriser, l'augmentation et/ou la stabilité et/ou l'activité biologique de ces formes complexes.

En particulier, la présente invention concerne un procédé de criblage in vitro ou ex vivo d'actifs anti-âge comprenant au moins les étapes consistant à :
a) mettre en contact, au moins un type cellulaire contenant au moins une forme complexe conforme à l'invention, avec au moins un actif anti-âge à tester, dans des conditions propices à l'expression de ladite forme complexe, et
b) déterminer une teneur de ladite forme complexe,
c) comparer ladite teneur déterminée à l'étape b) à une teneur de ladite forme complexe déterminée en absence de composé chimique ou biologique à tester, une augmentation en forme(s) complexe(s) conforme(s) à l'invention étant indicative d'un actif doté de propriétés anti-âge.

Ainsi, avantageusement, il peut être effectué en outre à l'issue de l'étape c) une étape de sélection du ou des actifs pour lesquels on observe une augmentation de la teneur en forme(s) complexe(s) conforme(s) à l'invention.

Le présent texte décrit en outre l'utilisation cosmétique d'une quantité efficace de forme(s) complexe(s) conforme(s) à l'invention, à titre d'agent utile pour prévenir et/ou traiter les signes de vieillissement cutané.

Le présent texte décrit l'utilisation d'une quantité efficace de forme(s) complexe(s) conforme(s) à l'invention, pour prévenir et/ou traiter un amincissement d'un épiderme et/ou une perte de fermeté, d'élasticité, de densité, et/ou de tonicité d'un épiderme et/ou la formation de rides et ridules.

Le présent texte décrit encore l'utilisation d'une quantité efficace de forme(s) complexe(s) conforme(s) à l'invention, ou d'au moins un agent modulateur de la formation de ladite (desdites) forme(s) complexe(s), pour la préparation et/ou l'amélioration d'un modèle cellulaire pluristratifié, en particulier un modèle de peau reconstruite.

Le présent texte décrit également un procédé de préparation d'une peau reconstruite isolée comprenant au moins l'étape consistant à mettre en contact au moins une forme complexe selon l'invention, avec des cellules susceptibles de générer une peau reconstruite isolée, et notamment des kératinocytes.

Selon un autre aspect, la présente invention concerne un procédé cosmétique in vitro ou ex vivo pour caractériser l'efficacité d'un traitement cosmétique ou thérapeutique visant à prévenir et/ou traiter les signes de vieillissement cutané liés au vieillissement chronologique chez un individu, comprenant au moins la caractérisation quantitative d'au moins une forme complexe selon l'invention, une augmentation en forme(s) complexe(s) étant indicative d'une efficacité dudit traitement des signes de vieillissement cutané.

La présente invention concerne par ailleurs un in vitro ou ex vivo, non thérapeutique, pour caractériser l'efficacité d'un traitement susceptible de prévenir et/ou traiter les signes du vieillissement chronologique cutané chez un individu, comprenant au moins les étapes consistant à :
i. Disposer d'au moins un premier échantillon de surface cutané représentatif dudit individu,
ii. Quantifier au niveau dudit échantillon notamment via une technique de dosage ELISA, au moins une forme complexe selon l'invention,
iii. Renouveler les étapes i. et ii. sur un deuxième échantillon de surface cutané représentatif dudit individu, et
iv. Comparer les résultats obtenus à l'issu des étapes ii. et iii., notamment afin d'en déduire une information relative à au moins un effet dudit traitement,
lesdits premier et second échantillons de surface cutané correspondant à des stades de traitement différents,
une augmentation en forme(s) complexe(s) étant indicative d'une efficacité dudit traitement des signes de vieillissement chronologique cutané. Selon encore un autre aspect, la présente invention concerne l'utilisation d'au moins une forme complexe selon l'invention, à titre d'outil de criblage de composés biologiques ou chimiques susceptibles de favoriser l'augmentation et/ou la stabilité et/ou l'activité biologique desdites formes complexes.

### Définition de « formes complexes » de la CLSP

Comme précisé précédemment, les formes complexes conformes à l'invention sont représentées par un polypeptide de séquence d'acides aminés codée par une séquence d'acides nucléiques représentée par SEQ ID NO 1, ou un analogue de celle-ci ou un fragment de celle-ci.

Une forme complexe de la CLSP peut notamment être un multimère dérivant de l'association d'au moins la protéine CLSP entière ou de l'un de ses fragments avec soit elle-même, soit l'un de ses fragments, ou encore avec au moins une protéine cible, voire avec au moins une structure protéique de type enveloppe cornifiée protéolysée dans le *stratum corneum.*

Les fragments de CLSP conformes à l'invention sont des fragments présentant une longueur et une activité propice à leur mise en oeuvre sous la forme d'un complexe selon l'invention.

Plus particulièrement, une forme complexe de la CLSP peut être un dimère, et encore plus particulièrement un homodimère de CLSP entières, ou de fragments de CLSP.

Selon un mode de réalisation particulier, un dimère conforme à l'invention peut comprendre un premier monomère représenté par la protéine CLSP entière ou l'un de ses fragments, et un second monomère représenté par une structure distincte de la CLSP entière ou de l'un de ses fragments.

A titre de monomère représenté par une structure distincte de la CLSP entière ou de l'un de ses fragments pouvant convenir à l'invention, peuvent être cités les protéines précités et les fragments de celles-ci aptes à se dimériser avec la CLSP entière ou l'un de ses fragments.

Selon un autre mode de réalisation particulier, une forme complexe convenant à l'invention peut comprendre un peptide issu de la CLSP et distinct de la séquence de la CLSP entière, en particulier distinct de la séquence d'acides aminés codée par une séquence d'acides nucléiques représentée par SEQ ID NO 1.

En particulier, un peptide issu de la CLSP convenant à l'invention peut être un fragment de la CLSP, et plus particulièrement un fragment de la protéine codée par une séquence d'acides nucléiques représentée par SEQ ID NO 1, comprenant une longueur suffisante et une activité biologique propices à sa mise en oeuvre sous la forme d'un complexe selon l'invention.

Au sens de la présente invention, on entend désigner par « fragment de séquence d'acides nucléiques », une séquence d'acides nucléiques codant en partie pour les formes complexes conformes à l'invention, ou un analogue de celui-ci, et en particulier une séquence d'acides nucléiques représentée par SEQ ID NO 1 ou un analogue de celle-ci.

Par « analogue d'une séquence d'acides nucléiques », on entend désigner toute séquence d'acides nucléiques, éventuellement résultant de la dégénérescence du code des acides nucléiques, et codant pour un au moins une partie des formes complexes de séquence identique ou analogue à celle du polypeptide codé par ladite séquence d'acides nucléiques.

Les séquences d'acides nucléiques peuvent être issues de toutes origines possibles, à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

Selon une variante de réalisation, les formes complexes selon l'invention peuvent être solubles.

Au sens de la présente invention, le terme « soluble » entend qualifier la faculté de la forme peptidique complexée et considérée selon l'invention, à se solubiliser dans l'eau ou dans un milieu aqueux sans substances dénaturante des protéines de type agent chaotropique ou détergents ioniques, par exemple par opposition à la CLSP native, uniquement extractible en présence de tels agents.

Selon un autre mode de réalisation, les formes complexes conformes à l'invention dérivent au moins en partie d'un polypeptide dont la séquence d'acides aminés est représentée en tout ou partie par SEQ ID NO 2, ou un analogue de celle-ci, ou un fragment de celle-ci.

Au sens de la présente invention, on entend désigner de manière générale, sauf indication contraire, par la CLSP la séquence (SEQ ID NO 2) de la protéine ayant ou non subi des modifications post-traductionnelles de type glycosylation sur les résidus asparagine en position 25 et/ou 43, de type phosphorylation et/ou de type myristylation, susceptibles de modifier son poids moléculaire apparent ou sont point isoélectrique.

Il est par ailleurs connu que la séquence primaire d'un polypeptide, c'est-à-dire l'enchaînement de ses acides aminés, détermine des sites spécifiquement reconnus par des enzymes de type protéase, telles que la trypsine qui, une fois la reconnaissance de ces sites effective, vont induire le clivage du polypeptide par protéolyse. Cette protéolyse résulte en la génération de divers peptides, ou fragments de protéolyse, des formes complexes de la CLSP.

Les inventeurs ont détecté la présence de tels peptides dans le *stratum corneum.*

En conséquence, l'invention s'étend aux formes complexes comprenant au moins un fragment de protéolyse de la CLSP de séquence peptidique SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 et SEQ ID NO 25.

Ainsi, selon un mode de réalisation particulier, les formes complexes conformes à l'invention dérivent au moins d'un polypeptide dont la séquence d'acides aminés est représentée en tout ou partie par une séquence d'acides aminés choisie parmi les SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 et SEQ ID NO 25, et leurs mélanges.

Par « analogue d'un polypeptide », on entend désigner tout polypeptide présentant une homologie de séquence, en particulier vis-à-vis d'une des séquences caractéristiques dudit polypeptide, ainsi qu'une activité biologique de même nature.

Cet analogue peut être un agent peptidomimétique.

L'homologie peut être d'au moins 85 %, par exemple d'au moins 90 %, et par exemple d'au moins 95 %. L'homologie peut être déterminée par comparaison visuelle ou au moyen de tout outil informatique généralement utilisé dans le domaine, tels que les programmes BLAST disponibles sur www.ncbi.nlm.nih.gov et utilisés avec les paramètres configurés par défaut.

L'homologie de séquence peut résulter de modifications issues de mutation ou de variation dans les séquences des peptides selon l'invention provenant soit de la délétion d'un ou plusieurs acides aminés, ou de l'insertion d'un ou plusieurs acides aminés, soit de la substitution d'un ou plusieurs acides aminés dans les séquences caractéristiques d'un polypeptide selon l'invention.

Au sens de l'invention, on entend désigner par « fragment » toute portion peptidique, comprenant au moins 4, au moins 6, en particulier au moins 8, et plus particulièrement au moins 12 acides aminés consécutifs de la protéine CLSP, et une activité biologique sensiblement similaire.

De manière générale, les analogues polypeptidiques peuvent comprendre des substitutions conservatrices par rapport à la séquence d'acides aminés naturelle.

Plusieurs de ces modifications peuvent être combinées.

A titre d'exemple de mutations que l'on peut considérer dans la présente invention, il peut être mentionné, de manière non exhaustive, le remplacement d'un ou plusieurs résidus d'acides aminés par des résidus d'acides aminés ayant un indice hydropathique similaire sans pour autant affecter de manière sensible les propriétés biologiques des formes complexes selon l'invention .

L'indice hydropathique est un indice attribué aux acides aminés en fonction de leur hydrophobicité et de leur charge (Kyte et al. (1982), J. Mol. Biol., 157 : 105).

Les formes complexes également visées par la présente invention peuvent dériver de polypeptides tels que définis ci-dessus ayant subi une ou plusieurs modification(s) post-traductionnelle(s).

Par « modification(s) post-traductionnelle(s) », on entend englober l'ensemble des modifications qu'un peptide ou une protéine est susceptible de subir à l'issue de sa synthèse dans une cellule, telle que par exemple une ou des phosphorylation(s), une ou des thiolation(s), une ou des acétylation(s), une ou des glycosylation(s), une ou des lipidation(s), telles qu'une myristylation, un réarrangement structural de type formation de ponts disulfures et/ou de type clivage à l'intérieur de la séquence peptidique.

L'analogue présente, par ailleurs, sensiblement la même activité biologique que le polypeptide naturel.

Selon un mode de réalisation, les formes complexes convenant à la mise en oeuvre de l'invention peuvent également dériver de polypeptides naturels ou synthétiques, le cas échéant susceptibles d'être obtenus après lyse enzymatique ou chimique de la forme native de la CLSP ou par synthèse chimique ou biologique ou par extraction à partir d'un tissu biologique, comme par exemple la peau, exprimant naturellement ces formes complexes, ainsi que les différentes formes post-traductionnelles de celle-ci.

L'homme de l'art peut obtenir les formes complexes conformes à l'invention au moyen de procédés à base d'ADN recombinant, comme par exemple, ceux décrits dans le manuel « Molecular Cloning - A Laboratory Manual » (2ème édition), Sambrook et al., 1989, Vol. I-III, Coldspring Harbor Laboratory, Coldspring Harbor Press, NY, (Sambrook).

Selon un autre mode de réalisation, les formes complexes convenant à la mise en oeuvre de l'invention peuvent également être fusionnées avec un autre polypeptide distinct de ceux identifiés précédemment, un agent de ciblage hydrophile ou hydrophobe, un précurseur de bio-conversion, un agent de marquage luminescent, radioactif ou colorimétrique.

De manière non limitative, on peut citer comme exemple de composés susceptibles d'être couplés avec les formes complexes conformes à l'invention des protéines fluorescentes comme la « Green Fluorescent Protein », des composés chimiques fluorescents tels que la rhodamine, la fluorescéine, ou le Texas Red®, des composés phosphorescents, des éléments radioactifs, tels que ³H, ¹⁴C, ³⁵S, ¹²¹I ou ¹²⁵I, ou des agents de marquage colorimétrique comme les substrats chromogènes sensibles à l'action de la galactosidase, de la peroxydase, de la chloramphénicol acétyltransférase, de la luciférase ou de la phosphatase alcaline.

Selon la nature des composés susceptibles d'être couplés avec les formes complexes conformes à l'invention, le couplage peut être opéré par des procédés chimiques, notamment au moyen de fonctions chimiques réactives ou par des procédés de biologie moléculaire connus de l'homme de l'art.

A titre de méthodes de détection d'un polypeptide, il peut être fait mention du Western-blot, du Slot-blot, du Dot-blot, des méthodes ELISA (Enzyme Linked Immuno-Sorbent Assay) de type singleplex ou multiplex, des méthodes de protéomique ou de glycomique, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocale, de FRET (fluorescence resonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie en temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiment), des méthodes par gène rapporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes de focalisation isoélectrique, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen).

Comme il ressort de ce qui suit, la détection des formes complexes selon l'invention est privilégiée selon une méthode ELISA.

### Utilisation des formes complexes selon l'invention, à des fins de diagnostic d'un état de l'épiderme et/ou de criblage d'actifs anti-âge.

Comme précisé précédemment, la présente invention concerne selon un autre de ses aspects des procédés non invasifs pour caractériser, notamment de façon *in vitro* ou *ex vivo,* l'état de surface d'un épiderme non pathologique ou encore l'efficacité d'un traitement cosmétique ou thérapeutique visant à caractériser qualitativement ou quantitativement l'expression des formes complexes formées au moins en partie de la CLSP, de l'un de ses dérivés ou fragments.

Ces procédés sont particulièrement avantageux dans la mesure où leur mise en oeuvre ne nécessite pas de recourir obligatoirement à une technique opératoire pour procéder à une telle caractérisation.

Les procédés selon l'invention, décrits ci-après, peuvent être effectués sur un échantillon, par exemple isolé, d'épithélium, et notamment d'épiderme, prélevé chez un individu.

Les procédé selon l'invention peuvent également être effectués sur un échantillon d'épithélium, et notamment d'épiderme, prélevé à partir d'un modèle cellulaire épithélial, et notamment épidermique, ou d'une peau isolée reconstruite afin d'en qualifier l'état.

Un extrait de l'épiderme peut ainsi être obtenu par simple stripping et directement analysé par la méthode ELISA telle que décrite dans l'exemple 1, ci-après.

La technique stripping consiste à appliquer une surface collante à la surface de l'épiderme comme le Blenderm® de 3M, le D'squam (adhésif commercial de CuDERM), la colle cyanoacrylate. Grâce à ces strippings, les cornéocytes adhérents et le contenu de leurs espaces intercellulaires peuvent être prélevés et soumis par la suite à une extraction permettant d'avoir accès au contenu protéique.

Le prélèvement d'un échantillon convenant au procédé peut aussi être effectué de façon plus directe par « lavages » de la surface cutanée au moyen par exemple d'accessoires de type turbine à ailette, de type cellule à spirale, tel que décrit dans le brevet FR 2 667 778 associée à un circuit fluidique, ou simplement par ajout/prélèvement d'une goutte de tampon à la surface de la peau.

A titre indicatif, d'autres méthodes de prélèvement adaptées à la mise en oeuvre de l'invention, on peut mentionner des méthodes par grattage de la partie supérieure du *stratum corneum* au moyen d'un système bilames ou par shave biopsie. Cette technique permet de recueillir des squames qui peuvent ensuite être directement analysées par différentes techniques pour déterminer les taux en minéraux, aminoacides ou lipides.

A l'issu du prélèvement, l'échantillon est caractérisé par la méthode ELISA.

Cette méthode repose notamment sur la mise en oeuvre d'anticorps appropriés à la détection de formes complexes considérées selon l'invention. Un anticorps susceptible d'être utilisé à titre d'outil d'évaluation d'un état d'un épiderme peut être obtenu par tout procédé connu de l'homme de l'art, tel que décrit dans « Antibodies: A Laboratory Manual », Cold Spring Harbor Laboratory Press, Cold Spring Harbor, N.Y. (1990).

Telle que décrite dans l'exemple 1 suivant, la méthode de détection des formes complexes de la CLSP nécessite notamment l'utilisation de deux types d'anticorps monoclonaux, développés par la société covalAb, à savoir un anticorps de capture (clone DB15C9) et un anticorps de sélection marqué au « sulfo-tag » (CLSP clone DB7g12).

### Procédé de diagnostic d'un état de l'épiderme

Selon un mode de réalisation, un procédé de in vitro ou ex vivo d'un état d'un épithélium, par exemple un épiderme, comprend au moins les étapes consistant à :
a) déterminer, dans un échantillon de surface dudit épithélium, une teneur en au moins une forme complexe conforme à l'invention, et
b) comparer ladite teneur déterminée à l'étape a) à une valeur de référence.

Avantageusement, un procédé de l'invention est non invasif.

Une valeur de référence peut être, par exemple, une teneur en formes complexes déterminée sur un échantillon d'épiderme prélevé sur un épithélium, et notamment une peau normale, c'est-à-dire satisfaisante sur le plan physiologique, à l'image par exemple d'une peau jeune.

La mesure d'une valeur de référence peut être effectuée parallèlement ou séquentiellement à la détermination de ladite teneur desdites formes complexes.

Une comparaison d'une teneur déterminée avec une valeur de référence peut permettre d'évaluer une déviation par rapport à cette valeur.

L'analyse de l'intensité et/ou de la nature de cette déviation (négative ou positive) peut être informative de l'état de l'épiderme.

La caractérisation d'un état d'un épiderme peut être indicative d'un éventuel trouble cutané qui peut être corrigé par l'utilisation de composés susceptibles de moduler l'expression des formes complexes considérées dans l'invention.

Ainsi, une diminution en forme(s) complexe(s) de l'invention est indicative d'un état de vieillissement chronologique.

Un procédé selon l'invention peut être mis en oeuvre dans un procédé de diagnostic *in vitro* ou *ex vivo* d'un état physiologique d'un épithélium, et notamment de l'épiderme, en vue en particulier d'apprécier son état de vieillissement chronobiologique et par exemple ses carences et/ou altérations associées chez un individu.

### Criblage de composés biologiques ou chimiques

La présente invention se rapporte à l'utilisation des formes complexes conformes à l'invention, à titre d'outil de criblage de composés biologiques ou chimiques susceptibles de favoriser l'augmentation et/ou la stabilité et/ou l'activité biologique de ces formes complexes.

La présente invention se rapporte également à un procédé de criblage in vitro ou ex vivo de d'actifs anti-âge comprenant au moins les étapes consistant à :
a) mettre en contact, avec au moins un actif anti-âge à tester, au moins un type cellulaire contenant au moins une forme complexe conforme à l'invention dans des conditions propices à l'expression de ladite forme complexe,
b) déterminer une teneur de ladite forme complexe, et
c) comparer ladite teneur déterminée à l'étape b) à une teneur de ladite forme complexe déterminée en absence de composé chimique ou biologique à tester.

La comparaison effectuée à l'étape c) peut permettre de déduire une information quant à la propriété dudit composé testé à moduler l'expression des formes complexes conformes à l'invention.

Comme précisé ci-dessus, ce procédé peut comprendre en outre une étape visant à retenir les actifs pour lesquels on observe une augmentation de la teneur en forme(s) complexe(s).

Plus particulièrement, le procédé utile pour caractériser l'efficacité d'un traitement susceptible de prévenir et/ou traiter les signes du vieillissement cutané chez un individu, peut comprendre au moins les étapes consistant à :
i. Disposer d'au moins un premier échantillon de surface cutané représentatif dudit individu,
ii. Quantifier au niveau dudit échantillon notamment via la technique de dosage ELISA exposée en exemple 1, au moins une forme complexe considérée dans la présente invention,
iii. Renouveler les étapes i. et ii. sur un deuxième échantillon de surface cutané représentatif dudit individu, et
iv. Comparer les résultats obtenus à l'issu des étapes ii. et iii., notamment afin d'en déduire une information relative à au moins un effet du traitement,
lesdits premier et second échantillons de surface cutané correspondant à des stades de traitement différents.

Dans l'hypothèse où une mesure de valeur de référence est effectuée préalablement à la mise en oeuvre du composé biologique ou chimique ou du facteur physico-chimique à tester, la méthode selon l'invention peut permettre en outre, le cas échéant, d'apprécier l'efficacité potentielle dudit composé.

Cette quantité peut ne pas être affectée par la présence dudit composé ou en revanche être inhibée ou stimulée.

Dans l'hypothèse où une augmentation est constatée, le composé testé est susceptible d'être utilisé par exemple à titre d'actif anti-âge.

Selon une variante de réalisation, les procédés tels que définis ci-dessus peuvent comprendre en outre au moins une étape consistant à administrer à un individu une composition de soin cosmétique établie ou sélectionnée au regard de l'information obtenue à l'issue de l'étape iv.

La détermination d'une teneur des formes complexes conformes à l'invention est effectuée au moyen d'une méthode ELISA, notamment celle spécifiquement décrite en exemple 1, ci-après.

Les formes complexes de la CLSP telles que décrites précédemment peuvent être utilisées dans une composition cosmétique ou thérapeutique.

Il est entendu que l'ensemble des compositions cosmétiques ou thérapeutiques considérées mettent en oeuvre un milieu physiologiquement acceptable.

Au sens de la présente invention, on entend désigner par « milieu physiologiquement acceptable », un milieu convenant à l'application d'une composition sur un épithélium ou une matière kératinique, telle que la peau, le cuir chevelu, les lèvres, les muqueuses et les fibres kératiniques comme les cheveux, les ongles et les poils, ou le cas échéant par voie orale ou parentérale.

Au sens de l'invention, on entend désigner par « cosmétique » une utilisation destinée, principalement, à procurer un effet esthétique et/ou du confort.

Au sens de la présente invention, on entend désigner par « thérapeutique » une composition pouvant être mise en oeuvre dans le cadre d'un traitement prophylactique et/ou curatif, ou d'une méthode d'évaluation d'un état d'un épithélium, et notamment de l'épiderme.

Par « prophylactique » ou « préventif » au sens de l'invention, on entend la diminution du risque de survenue d'un phénomène, par exemple une pathologie.

Selon un autre mode de réalisation, une composition cosmétique ou thérapeutique peut comprendre, en outre, au moins un agent actif cosmétique et/ou thérapeutique.

Comme exemples d'agents actifs utilisables dans le cadre de la présente invention, il peut être fait mention d'huiles cosmétiques, telles que les huiles de silicone, les huiles végétales de type triglycérides, les huiles hydrocarbonées telles que l'huile de Parleam et les esters d'acides gras et d'alcool gras.

D'autres agents actifs permettant d'améliorer l'état de la peau peuvent également être utilisé, tels que des actifs hydratants ou des agents actifs permettant d'améliorer la barrière lipidique naturelle, tels que des céramides, des sulfates de cholestérol et/ou des acides gras, et leurs mélanges.

Des enzymes ayant une activité sur la peau peuvent également être utilisées, telles que des protéases, des lipases, des glucosidases, des amidases, des cérébrosidases et/ou des mélanases, et leurs mélanges.

D'une manière générale, toute composition peut être appliquée sur la peau (sur toute zone cutanée du corps) ou sur les muqueuses (buccale, jugale, gingivale, génitale, conjonctivale, ...).

De façon connue, une composition cosmétique peut contenir également des adjuvants habituels dans le domaine cosmétique, tels que des gélifiants hydrophiles ou lipophiles, des additifs hydrophiles ou lipophiles, des conservateurs, des antioxydants, des solvants, des parfums, des charges, des filtres, des absorbeurs d'odeurs et des matières colorantes.

Les quantités des différents constituants des compositions sont celles classiquement utilisées dans les domaines considérés.

La quantité en forme(s) complexe(s) conforme(s) à l'invention contenue dans une composition, encore dite « quantité efficace » est, bien entendu, fonction de la nature du composé et de l'effet recherché et peut donc varier dans une large mesure.

Pour donner un ordre de grandeur, une composition peut contenir une forme complexe selon l'invention en une quantité représentant de 0,00001 % à 50 % du poids total de la composition, en particulier en une quantité représentant de 0,001 % à 10 % du poids total de la composition, et plus particulièrement en une quantité représentant de 0,1 % à 1 % du poids total de la composition.

Une composition peut être plus particulièrement dédiée à la réduction et/ou au traitement d'affections susceptibles de détériorer l'état d'un épithélium, et notamment d'un épiderme.

Un tel état peut être d'origine chronologique (i.e. lié au temps écoulé comme le vieillissement cutané) et/ou indicatif d'un trouble cutané, lié par exemple au photovieillissement.

Ainsi, une composition, notamment cosmétique, peut, en particulier, être destinée à prévenir et/ou traiter un amincissement d'un épiderme et/ou une perte de fermeté, d'élasticité, de densité et/ou de tonicité d'un épiderme et/ou la formation de rides et ridules.

Selon un autre mode de réalisation, une composition, en particulier cosmétique, peut, notamment, être destinée à prévenir et/ou à traiter des signes cutanés de sécheresse, en particulier à prévenir et/ou à traiter une déshydratation d'un épiderme.

Selon un autre mode de réalisation, une composition, notamment cosmétique, peut être destinée à prévenir et/ou à traiter des signes de vieillissement épidermique.

Selon un autre aspect, le présent texte se rapporte à un procédé de traitement cosmétique des signes de vieillissement cutané, comprenant au moins une étape consistant à appliquer sur une partie au moins de la peau, des muqueuses et/ou des fibres kératiniques, au moins une composition cosmétique conforme à l'invention.

Selon encore un autre aspect, le présent texte décrit l'utilisation, notamment cosmétique et/ou thérapeutique, de formes complexes de la protéine CLSP, de fragments peptidiques de cette protéine ou d'analogues de celle-ci, ou d'un agent modulateur de l'activité, de l'expression et/ou de la stabilité d'un tel polypeptide, notamment pour prévenir les signes de vieillissement cutané, notamment pour prévenir et/ou traiter les peaux âgées.

Le présent texte décrit également l'utilisation d'une quantité efficace de formes complexes conformes à l'invention pour la préparation et/ou l'amélioration d'un modèle cellulaire pluristratifié, notamment de type épidermique ou muqueux, et en particulier un modèle de peau reconstruite.

Au sens du texte, on entend désigner par « modèle de peau reconstruite », un modèle dans lequel sont associés différents types cellulaires, tels que notamment les constituants naturels de la peau, à l'image par exemple des kératinocytes, des fibroblastes, des cellules de Langerhans et des mélanocytes.

Les cellules de type fibroblastes peuvent être irradiées ou non.

De tels modèles et leur préparation sont connus de l'homme de l'art.

Ainsi, le présent texte décrit également un procédé de préparation d'une peau reconstruite isolée, comprenant au moins l'étape consistant à mettre en contact au moins une forme complexe selon l'invention, avec des cellules susceptibles de générer une peau reconstruite isolée, et notamment des kératinocytes.

Les exemples et figures qui suivent sont présentés à titre illustratif et non limitatif de l'invention.
**Figure 1** **:** elle illustre la relation entre l'âge et la quantité en formes complexes selon l'invention présente au niveau de la peau de l'avant-bras et du visage.
**Figure 2** **:** elle illustre les effets liés à l'application d'une composition cosmétique, composée à 10% de Bifidiobiotic (complexe CLR) et à 0,002% de Phytosphingosine-SLC (traitement « G »), sur la quantité en formes complexes selon l'invention, au niveau de la peau de l'avant-bras et du visage.

### Exemple 1 :

### Mise au point d'un test ELISA haute sensibilité permettant le dosage de formes complexes de la CLSP conformes à l'invention :

Des plaques CLSP développées à façon avec la technologie MesoScale Discovery (MSD) ont été utilisées dans cet essai. Les anticorps monoclonaux dirigés contre une protéine recombinante CLSP « full-length » fusionnée avec la GST ont été développés par la société CovalAb (Lyon, France) (Mehul et 2006). L'anticorps de capture (Clone DB15C9) a été déposé à une concentration de 200 µg/ml sur une plaque standard 96 puits de type « small spot » (MesoScale).

Une courbe de référence (5 à 0.078 µg/ml) a été établie utilisant la protéine recombinante CLSP.

Chaque plaque a été bloquée par un tampon de blocage (MesoScale) pendant 1 heure à température ambiante. 25 µl de chaque échantillon et de standards ont été déposés en triple et incubées sous agitation pendant 1 heure à température ambiante. La plaque a été alors lavée 4 fois au tampon Tris (MesoScale). Les plaques ont été alors incubées avec agitation pendant 1 heure à température ambiante avec 25 µl d'anticorps de détection (Mesoscale) marqué au « sulfo-tag » (CLSP clone DB7g12) à une concentration de 1 µg/ml. Les plaques ont été lavées 4 fois avec un tampon de Tris (MesoScale) avant l'addition de 150 µl de 1 X «read buffer T» (MesoScale). Les plaques ont été lues sur l'appareil « Sector Imager 6000 ». Les données ont été standardisées en termes de ng CLSP par µg de protéine totale.

### Exemple 2 :

### Caractérisation de la teneur en formes complexes CLSP dans différents échantillons prélevés par stripping.

Cette caractérisation est réalisée à l'aide du test ELISA décrit en exemple 1.

Deux études cliniques ont été menées : une étude connaissance (2 groupes de 20 sujets femmes, peau caucasienne : 30-35 ans et 60-65ans) avec des prélèvements sur l'avant-bras et le visage, et une étude produit sur 56 jours, soit 2 mois (1 groupe de 24 sujets femmes, peau caucasienne, âgées de 40-45 ans) avec des prélèvements effectués uniquement sur l'avant-bras.

Le produit est un sérum composé à 10 % de Bifidiobiotic (complexe CLR) et 0,002 % de Phytosphingosine-SLC (traitement « G »).

Le complexe CLR se rapporte à un lysat enregistré sous le nom INCI : Bifidat ferment Lysate, sous le nom EINECS : Bifidobacterium longum, sous le N° EINECS : N° 306-168-4 et sous le N° CAS : N° 96507-89-0.

Un tel lysat est notamment commercialisé sous la dénomination Repair Complex CLR® par la société K. RICHTER GmbH.

La dénomination Phytosphingosine-SLC correspond à un dérivé Phytosphingosine-salicylate commercialisé par la société EVONIK GOLDSCHMIDT.

Pour les deux études, des prélèvements D-Squame ont été fait au niveau du visage (joue) et de l'avant-bras (face postérieure). Les cornéodiscs D-Squame prélevés sur les avant-bras ont servis pour l'évaluation des effets produit.

### a) Extraction des protéines des cornéodiscs D-Squame :

Le cornéodisc est placé dans un tube Eppendorf 2 ml, face collante vers l'intérieur, auquel on ajoute 550 µl de tampon d'extraction dit «Native+» (TBS, 1 %Triton X-100, 1M NaCl) et une bille en acier inox par tube. Le tube est ensuite placé dans les portoirs du vibrobroyeur MM400 (Retsch). L'extraction se fait par vibrobroyage pendant un cycle de 2 min à 30 Htz. Le milieu est ensuite récupéré et filtré sur colonne Millipore Ultrafree de 0,45 µm suivi d'une centrifugation de 5 min à 5000 g à 4 °C. Le surnageant est conservé à -20 °C en attendant d'être analysé.

### b) Analyse statistique

L'analyse des biomarqueurs pour les effets âge (jeune *vs*. âgé) et zone (bras *vs*. visage) est réalisée à l'aide d'un modèle mixte d'analyse de la variance avec les facteurs âge, zone, et l'interaction âge*zone en effets fixes, et le facteur sujet en aléatoire. L'effet zone pour une classe d'âge et l'effet âge pour une zone ont été réalisés à l'aide de contrastes.

L'analyse des biomarqueurs, pour l'effet traitement, est réalisée à l'aide d'un modèle mixte d'analyse de la variance sur les différences appariées Actif - Placebo avec le facteur temps, en effet fixe, et le facteur sujet en effet aléatoire.

L'effet traitement à J0 et J56 est testé à l'aide de contrastes.

Les variables ont été, lorsque c'était nécessaire, préalablement log transformées pour rendre leur distribution plus Gaussienne.

On utilise le logiciel SPSS version 14 pour les analyses descriptives (graphiques des moyennes et box-plots), et le logiciel SAS Enterprise Guide version 3 pour la partie interférentielle.

Le risque alpha de première espèce a été fixé à 5 % dans une approche bilatérale.

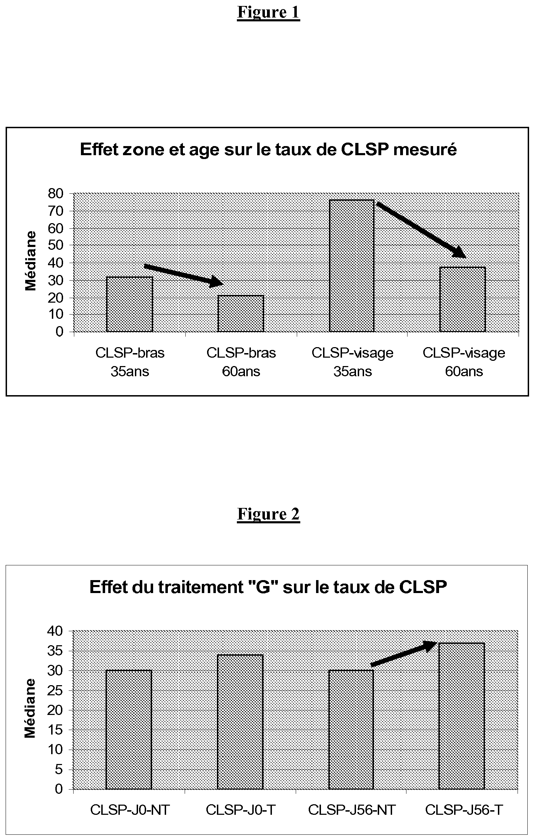

Les résultats de ces deux études sont illustrés en figures 1 et 2.

Les données révèlent une diminution des formes complexes de la CLSP avec l'âge, telles que représentées en figure 1.

Le kit ELISA haute sensibilité développé permet bien de vérifier que l'expression des formes complexes de la CLSP diminue avec l'âge.

Pour sa part, la figure 2 représente l'effet du produit testé sur la quantité en formes complexes. On constate manifestement une augmentation significative de la quantité en formes complexes, ce qui démontre l'effet activateur de ce produit. Ce dernier présente donc un réel effet anti-âge.

## Revendications

1. Utilisation d'au moins une forme complexe de la Calmoduline-like skin protein ou CLSP représentée par un polypeptide de séquence d'acides aminés codée par une séquence d'acides nucléiques représentée par SEQ ID NO 1, ou un analogue présentant une homologie de séquence d'au moins 85 % avec ledit polypeptide, ainsi qu'une activité biologique de même nature, ou un fragment de protéolyse de la CLSP de séquence peptidique choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO18, SEQ ID NO 19, SEQ ID NO 20, SEQ ID NO 21, SEQ ID NO 22, SEQ ID NO 23, SEQ ID NO 24 et SEQ ID NO 25, conjugué avec soit une protéine distincte de la CLSP, soit une autre protéine CLSP, soit un fragment de protéolyse de ladite autre protéine CLSP de séquence peptidique choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 et SEQ ID NO 25, à titre d'outil de caractérisation *in vitro* ou *ex vivo* d'un état de vieillissement chronologique d'un épithélium,
une diminution en forme(s) complexe(s) étant indicative d'un état de vieillissement chronologique.

2. Utilisation selon la revendication 1, dans laquelle le polypeptide a une séquence d'acides aminés représentée en tout ou partie par une séquence représentée par SEQ ID NO 2, ou un analogue de celle-ci présentant une homologie de séquence d'au moins 85 % avec ledit polypeptide, ainsi qu'une activité biologique de même nature.

3. Utilisation selon la revendication 1, dans laquelle le polypeptide a une séquence d'acides aminés représentée en tout ou partie par une séquence d'acides aminés choisie parmi les SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO18, SEQ ID NO 19, SEQ ID NO 20, SEQ ID NO 21, SEQ ID NO 22, SEQ ID NO 23, SEQ ID NO 24 et SEQ ID NO 25.

4. Utilisation selon l'une quelconque des revendications précédentes, **caractérisée en ce que** la protéine distincte dudit polypeptide est une protéine cible choisie parmi le polypeptide 14.3.3 Beta, le 14.3.3 Sigma, les annexines II et V, les calréticulines, l'ERp72, la Nucléoline, la Transglutaminase 3, la MAGED1 (Melanoma antigen gene D1), le PPP4C (Protein phosphatase 4) et l'USF2 (Upstream transcription factor 2), et le motif YWHAQ (14.3.3 Tau).

5. Procédé, in vitro ou ex vivo, de caractérisation d'un état d'un épithélium comprenant au moins les étapes consistant à :
a) déterminer dans un échantillon de surface dudit épithélium, une teneur en au moins une forme complexe telle que définie selon l'une des revendications 1 à 4, et
b) comparer ladite teneur déterminée à l'étape a) à une valeur de référence,
une diminution en forme(s) complexe(s) étant indicative d'un état de vieillissement chronologique.

6. Procédé de criblage in vitro ou ex vivo d'actifs anti-âge comprenant au moins les étapes consistant à :
a) mettre en contact, au moins un type cellulaire contenant au moins une forme complexe telle que définie selon l'une des revendications 1 à 4, avec au moins un actif anti-âge à tester, dans des conditions propices à l'expression de ladite forme complexe,
b) déterminer une teneur de ladite forme complexe, et
c) comparer ladite teneur déterminée à l'étape b) à une teneur de ladite forme complexe déterminée en absence de composé chimique ou biologique à tester,
une augmentation en forme(s) complexe(s) telle que définie selon l'une des revendications 1 à 4 étant indicative d'un actif doté de propriétés anti-âge.

7. Procédé cosmétique in vitro ou ex vivo pour caractériser l'efficacité d'un traitement cosmétique ou thérapeutique visant à prévenir et/ou traiter les signes de vieillissement cutané liés au vieillissement chronologique chez un individu, comprenant au moins la caractérisation quantitative d'au moins une forme complexe telle que définie selon l'une des revendications 1 à 4,
une augmentation en forme(s) complexe(s) étant indicative d'une efficacité dudit traitement des signes de vieillissement cutané.

8. Procédé in vitro ou ex vivo, non thérapeutique, pour caractériser l'efficacité d'un traitement susceptible de prévenir et/ou traiter les signes du vieillissement chronologique cutané chez un individu, comprenant au moins les étapes consistant à :
i. Disposer d'au moins un premier échantillon de surface cutané représentatif dudit individu,
ii. Quantifier au niveau dudit échantillon notamment via une technique de dosage ELISA, au moins une forme complexe telle que définie selon l'une quelconque des revendications 1 à 4,
iii. Renouveler les étapes i. et ii. sur un deuxième échantillon de surface cutané représentatif dudit individu, et
iv. Comparer les résultats obtenus à l'issu des étapes ii. et iii., notamment afin d'en déduire une information relative à au moins un effet dudit traitement,
lesdits premier et second échantillons de surface cutané correspondant à des stades de traitement différents,
une augmentation en forme(s) complexe(s) étant indicative d'une efficacité dudit traitement des signes de vieillissement chronologique cutané.

9. Utilisation d'au moins une forme complexe telle que définie selon l'une des revendications 1 à 4, à titre d'outil de criblage de composés biologiques ou chimiques susceptibles de favoriser l'augmentation et/ou la stabilité et/ou l'activité biologique desdites formes complexes.

## Patentansprüche

1. Verwendung von mindestens einer komplexen Form des Calmodulin-artigen Hautproteins oder CLSP, das durch ein Polypeptid der Aminosäuresequenz dargestellt ist, die durch eine Nukleinsäuresequenz kodiert wird, die durch SEQ 1D NO 1 dargestellt ist, oder ein Analog, das eine Sequenzhomologie von mindestens 85 % mit dem Polypeptid, sowie eine biologische Aktivität der gleichen Art aufweist, oder eines Proteolysefragments des CLSP der Peptidsequenz, die ausgewählt ist aus SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID N018, SEQ ID NO 19, SEQ ID NO 20, SEQ ID NO 21, SEQ ID NO 22, SEQ ID NO 23, SEQ ID NO 24 und SEQ ID NO 25, konjugiert entweder mit einem von CLSP unterschiedlichen Protein oder einem anderen CLSP-Protein, und zwar: einem Proteolysefragment des anderen CLSP-Proteins der Peptidsequenz, die ausgewählt ist aus SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24 und SEQ ID NO 25, als Werkzeug zur Charakterisierung, in vitro oder ex vivo, eines chronologischen Alterungszustands eines Epithels, wobei eine Abnahme an komplexen Formen(en) ein Hinweis ist auf einen chronologischen Alterungszustand eines Epithels.

2. Verwendung nach Anspruch 1, wobei das Polypeptid eine Aminosäuresequenz, die vollständig oder ganz durch eine Sequenz dargestellt ist, die durch SEQ ID NO 2 dargestellt ist, oder ein Analog davon aufweist, das eine Sequenzhomologie von mindestens 85 % mit dem Polypeptid sowie eine biologische Aktivität der gleichen Art aufweist.

3. Verwendung nach Anspruch 1, wobei das Polypeptid eine Aminosäuresequenz aufweist, die ganz oder teilweise durch eine Aminosäuresequenz dargestellt ist, die ausgewählt ist SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24 und SEQ ID NO 25.

4. Verwendung nach einem der vorangehenden Ansprüche, **dadurch gekennzeichnet, dass** das von dem Polypeptid unterschiedliche Protein ein Zielprotein ist, das ausgewählt ist aus Polypeptid beta 14.3.3, Polypeptid sigma 14.3.3, den Annexinen II und V, Calreticulinen, ERp72, Nucleolin, Transglutaminase 3, MAGED1 (Melanom-Antigen-Gen Dl), PPP4C (Proteinphosphatase 4) und USF2 (Stromaufwärts-Transkriptionsfaktor 2) und der Einheit YWHAQ (14.3.3 Tau).

5. In-vitro- oder Ex-vivo-Verfahren zur Charakterisierung eines Zustands eines Epithels, umfassend mindestens die folgenden Schritte:
a) Bestimmen, in einer Oberflächenprobe des Epithels, eines Gehalts an mindestens einer komplexen Form, wie nach einem der Ansprüche 1 bis 4 definiert, und
b) Vergleichen des in Schritt a) bestimmten Gehalts mit einem Referenzwert, wobei eine Verringerung an komplexen Form(en) ein Hinweis auf einen chronologischen Alterungszustand ist.

6. In-vitro- oder Ex-vivo-Screeningverfahren von Anti-Aging-Wirkstoffen, umfassen mindestens die folgenden Schritte:
a) Inkontaktbringen mindestens eines Zelltyps, enthaltend mindestens eine komplexe Form, wie nach einem der Ansprüche 1 bis 4 definiert, mit mindestens einem zu testenden Anti-Aging-Wirkstoff unter Bedingungen, die zur Expression der komplexen Form geeignet sind,
b) Bestimmen eines Gehalts der komplexen Form, und
c) Vergleichen des in Schritt b) bestimmten Gehalts mit einem Gehalt der komplexen Form, der in Abwesenheit von zu testender chemischer oder biologischer Verbindung bestimmt wird.
wobei ein Anstieg an komplexen Form(en), wie nach einem der Ansprüche 1 bis 4 definiert, ein Hinweis ist auf einen mit Anti-Aging-Eigenschaften bereitgestellten Wirkstoff.

7. Kosmetisches In-vitro- oder Ex-vivo-Verfahren zur Charakterisierung der Wirksamkeit einer kosmetischen oder therapeutischen Behandlung mit dem Ziel der Verhinderung und/oder Behandlung der Hautalterungsanzeichen, die mit der chronologischen Alterung bei einem Individuum zusammenhängen, umfassend mindestens die quantitative Charakterisierung von mindestens einer komplexen Form, wie nach einem der Ansprüche 1 bis 4 definiert, wobei ein Anstieg an komplexen Form(en) ein Hinweis ist auf eine Wirksamkeit der Behandlung der Hautalterungsanzeichen.

8. Nicht therapeutisches In-vitro- oder Ex-vivo-Verfahren zur Charakterisierung der Wirksamkeit einer Behandlung, die zur Verhinderung und/oder Behandlung der Anzeichen der chronologischen Hautalterung bei einem Individuum geeignet ist, umfassend mindestens die folgenden Schritte:
i. Bereitstellen von mindestens einer ersten für das Individuum repräsentativen Hautoberflächenprobe,
ii. Quantifizieren in der Probe, insbesondere über ein ELISA-Testverfahren, mindestens einer komplexen Form wie nach einem der Ansprüche 1 bis 4 definiert,
iii. Wiederholen der Schritte i. und ii. mit einer zweiten für das Individuum repräsentativen Hautoberflächenprobe, und
iv. Vergleichen der in den Schritten ii. und iii., erhaltenen Ergebnisse, um eine Information bezüglich mindestens einer Wirkung der Behandlung abzuleiten,
wobei die ersten und zweiten Hautoberflächenproben verschiedenen Behandlungsstadien entsprechen,
ein Anstieg an komplexen Form(en) ein Hinweis ist auf eine Wirksamkeit der Behandlung der Anzeichen von chronologischer Hautalterung.

9. Verwendung von mindestens einer komplexen Form wie nach einem der Ansprüche 1 bis 4 definiert als Screeningwerkzeug von biologischen oder chemischen Verbindungen, die zur Begünstigung des Anstiegs und/oder der Stabilität und/oder der biologischen Aktivität der komplexen Formen geeignet sind.

## Claims

1. Use of at least one complex form of the Calmodulin-like skin protein (CLSP) represented by an amino acid sequence polypeptide encoded by a nucleic acid sequence represented by SEQ ID NO 1, or an analog showing a sequence homology of at least 85% with the said polypeptide, as well as a biological activity of the same nature, or a proteolysis fragment of the CLSP of a peptide sequence chosen from among SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, and SEQ ID NO 25, conjugated with either a protein distinct from CLSP, or another CLSP protein, or a proteolysis fragment of the other said CLSP peptide sequence protein selected from among SEQ ID NO 3, SEQ ID NO 4, SEQ ID NO 5, SEQ ID NO 6, SEQ ID NO 7, SEQ ID NO 8, SEQ ID NO: 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24 and SEQ ID NO 25, as an *in vitro or ex vivo* characterization tool of a chronological aging condition of an epithelium,
wherein a decrease in complex form(s) is indicative of a state of chronological aging.

2. Use according to claim 1, wherein the polypeptide has an amino acid sequence represented in whole or in part by a sequence represented by SEQ ID NO 2, or an analog thereof having a sequence homology of at least 85% with the said polypeptide, as well as a biological activity of the same nature.

3. Use according to claim 1, wherein the polypeptide has an amino acid sequence represented in whole or in part by an amino acid sequence selected from among SEQ ID NO 3, SEQ ID NO 4, SEQ ID NO 5 , SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, and SEQ ID NO 25.

4. Use according to any one of the preceding claims, **characterized in that** the distinct protein of the said polypeptide is a target protein selected from among 14.3.3 beta polypeptide, 14.3.3 sigma polypeptide, annexins II and V; calreticulans, ERp72, nucleolin, transglutaminase 3, MAGED1 (melanoma antigen gene D1), PPP4C (protein phosphatase 4) and USF2 (upstream transcription factor 2), and the motif YWHAQ (14.3.3 Tau).

5. *In vitro or ex vivo* method for characterizing a state of an epithelium comprising at least the steps of:
a) determining in a surface sample of the said epithelium, a content of at least one complex form as defined according to one of the claims 1 to 4, and
b) comparing the said determined content in step a) with a reference value,
wherein a decrease in complex form(s) is indicative of a condition of chronological aging.

6. *In vitro or ex vivo* method for screening of anti-aging agents comprising, at least, the steps of:
a) contacting at least one cell type containing at least one complex form as defined according to one of the claims 1 to 4, with at least one anti-aging active agent to be tested, under conditions conducive to the expression of the said complex form,
b) determining a content of the complex form, and
c) comparing the said content determined in step b) with a content of the said complex form determined in the absence of a chemical or biological compound to be tested, wherein an increase in complex form(s) as defined according to one of the claims 1 to 4 is indicative of an active agent possessing anti-aging properties.

7. *In vitro or ex vivo* cosmetic process to characterize the effectiveness of a cosmetic or therapeutic treatment aiming at preventing and/or treating the signs of skin aging related to chronological aging in an individual, comprising at least the quantitative characterization of at least one complex form as defined in any one of the claims 1 to 4,
wherein an increase in complex form(s) is indicative of an efficacy of the said treatment for signs of skin aging.

8. *In vitro or ex vivo,* non-therapeutic method to characterize the effectiveness of a treatment likely to prevent and/or treat signs of chronological aging in an individual, comprising at least the steps of:
i. Having at least a first representative skin surface sample of the said individual,
ii. Quantifying the said sample, particularly via an ELISA assay technique, with respect to at least one complex form as defined according to any one of the claims 1 to 4,
iii. Carrying out the steps i. and ii. on a second representative skin surface sample of the said individual, and
iv. Comparison of the results obtained at the end of the steps ii and iii, in particular to deduce information relating to at least one effect of the said treatment,
wherein the said first and second skin surface samples correspond to different stages of treatment, and
wherein an increase in complex form(s) is indicative of an efficacy of the said treatment of the signs of chronological aging of the skin.

9. Use of at least one complex form as defined in one of the claims 1 to 4, as a tool for screening for biological or chemical compounds capable of promoting the increase and/or stability and/or biological activity of the said complex forms.


### SEQUENCE LISTING

<110> L'OREAL
<120> utilisation de formes complexes de la protéine de type calmodulin-like
   skin protein CLSP
<130> BR87468/96081/KLP/PLC/se
<160> 25
<170> PatentIn version 3.3
<210> 1
   <211> 858
   <212> DNA
   <213> Homo Sapiens
<400> 1
   aattcccgga tccctgcggc tgcctgcact ctggaccacg agctctgaga gcagcaggtt      60
   gagggccggt gggcagcagc tcggaggctc cgcgaggtgc aggagacgca ggcatggccg     120
   gtgagctgac tcctgaggag gaggcccagt acaaaaaggc tttctccgcg gttgacacgg     180
   atggaaacgg caccatcaat gcccaggagc tgggcgcggc gctgaaggcc acgggcaaga     240
   acctctcgga ggcccagcta aggaaactca tctccgaggt tgacagcgac ggcgacggcg     300
   aaatcagctt ccaggagttc ctgacggcgg caaggaaggc cagggccggc ctggaggacc     360
   tgcaggtcgc cttccgcgcc ttcgaccagg atggcgacgg ccacatcacc gtggacgagc     420
   tcaggcgggc catggcgggg ctggggcagc cgctgccgca ggaggagctg gacgccatga     480
   tccgcgaggc cgacgtggac caggacgggc gggtgaacta cgaggagttc gcgaggatgc     540
   tcgcccagga gtgaggctcc ccgcctgtgt ccccctggct gcgctctgag ccttcagggc     600
   caccgcccgc tgctgctttt gtgctgggac tctccgggga aacctggtcg gtggatggga     660
   aactgcctcc ccctgggagg aaggctttgc gctccggggc ctggatgcgg cgccctcggg     720
   ccgcctgcga gcccctctct gccttcagac cttgggcaga aggaggcctc cttgggcctg     780
   gtcccccttt gccctgcagt ggaatgaggg ccccttaacc ccgcattgat ctaaataaag     840
   gactgccgag ttccaaaa                                                  858
<210> 2
   <211> 146
   <212> PRT
   <213> Homo Sapiens
<400> 2
   Met Ala Gly Glu Leu Thr Pro Glu Glu Glu Ala Gln Tyr Lys Lys Ala
   1               5                   10                  15
   Phe Ser Ala Val Asp Thr Asp Gly Asn Gly Thr Ile Asn Ala Gln Glu
               20                  25                  30
   Leu Gly Ala Ala Leu Lys Ala Thr Gly Lys Asn Leu Ser Glu Ala Gln
           35                  40                  45
   Leu Arg Lys Leu Ile Ser Glu Val Asp Ser Asp Gly Asp Gly Glu Ile
       50                  55                  60
   Ser Phe Gln Glu Phe Leu Thr Ala Ala Arg Lys Ala Arg Ala Gly Leu
   65                  70                  75                  80
   Glu Asp Leu Gln Val Ala Phe Arg Ala Phe Asp Gln Asp Gly Asp Gly
                   85                  90                  95
   His Ile Thr Val Asp Glu Leu Arg Arg Ala Met Ala Gly Leu Gly Gln
               100                 105                 110
   Pro Leu Pro Gln Glu Glu Leu Asp Ala Met Ile Arg Glu Ala Asp Val
           115                 120                 125
   Asp Gln Asp Gly Arg Val Asn Tyr Glu Glu Phe Ala Arg Met Leu Ala
       130                 135                 140
   Gln Glu
   145
<210> 3
   <211> 16
   <212> PRT
   <213> Homo Sapiens
<400> 3
   Ala Phe Asp Gln Asp Gly Asp Gly His Ile Thr Val Asp Glu Leu Arg
   1               5                   10                  15
<210> 4
   <211> 17
   <212> PRT
   <213> Homo Sapiens
<400> 4
   Ala Phe Asp Gln Asp Gly Asp Gly His Ile Thr Val Asp Glu Leu Arg
   1               5                   10                  15
   Arg
<210> 5
   <211> 16
   <212> PRT
   <213> Homo Sapiens
<400> 5
   Ala Phe Ser Ala Val Asp Thr Asp Gly Asn Gly Thr Ile Asn Ala Gln
   1               5                   10                  15
<210> 6
   <211> 23
   <212> PRT
   <213> Homo Sapiens
<400> 6
   Ala Phe Ser Ala Val Asp Thr Asp Gly Asn Gly Thr Ile Asn Ala Gln
   1               5                   10                  15
   Glu Leu Gly Ala Ala Leu Lys
               20
<210> 7
   <211> 13
   <212> PRT
   <213> Homo Sapiens
<400> 7
   Ala Gly Glu Leu Thr Pro Glu Glu Glu Ala Gln Tyr Lys
   1               5                   10
<210> 8
   <211> 14
   <212> PRT
   <213> Homo Sapiens
<400> 8
   Ala Gly Glu Leu Thr Pro Glu Glu Glu Ala Gln Tyr Lys Lys
   1               5                   10
<210> 9
   <211> 11
   <212> PRT
   <213> Homo Sapiens
<400> 9
   Ala Gly Leu Glu Asp Leu Gln Val Ala Phe Arg
   1               5                   10
<210> 10
   <211> 19
   <212> PRT
   <213> Homo Sapiens
<400> 10
   Ala Met Ala Gly Leu Gly Gln Pro Leu Pro Gln Glu Glu Leu Asp Ala
   1               5                   10                  15
   Met Ile Arg
<210> 11
   <211> 9
   <212> PRT
   <213> Homo Sapiens
<400> 11
   Glu Ala Asp Val Asp Gln Asp Gly Arg
   1               5
<210> 12
   <211> 24
   <212> PRT
   <213> Homo Sapiens
<400> 12
   Lys Ala Phe Ser Ala Val Asp Thr Asp Gly Asn Gly Thr Ile Asn Ala
   1               5                   10                  15
   Gln Glu Leu Gly Ala Ala Leu Lys
               20
<210> 13
   <211> 24
   <212> PRT
   <213> Homo Sapiens
<400> 13
   Lys Leu Ile Ser Glu Val Asp Gly Asp Gly Asp Gly Glu Ile Ser Phe
   1               5                   10                  15
   Gln Glu Phe Leu Thr Ala Ala Arg
               20
<210> 14
   <211> 15
   <212> PRT
   <213> Homo Sapiens
<400> 14
   Lys Leu Ile Ser Glu Val Asp Ser Asp Gly Asp Gly Glu Ile Ser
   1               5                   10                  15
<210> 15
   <211> 24
   <212> PRT
   <213> Homo Sapiens
<400> 15
   Lys Leu Ile Ser Glu Val Asp Ser Asp Gly Asp Gly Glu Ile Ser Phe
   1 5 10 15
   Gln Glu Phe Leu Thr Ala Ala Lys
   20
<210> 16
   <211> 24
   <212> PRT
   <213> Homo Sapiens
<400> 16
   Lys Leu Ile Ser Glu Val Asp Ser Asp Gly Asp Gly Glu Ile Ser Phe
   1 5 10 15
   Gln Glu Phe Leu Thr Ala Ala Arg
   20
<210> 17
   <211> 23
   <212> PRT
   <213> Homo Sapiens
<400> 17
   Leu Ile Ser Glu Val Asp Gly Asp Gly Asp Gly Glu Ile Ser Phe Gln
   1 5 10 15
   Glu Phe Leu Thr Ala Ala Arg
   20
<210> 18
   <211> 12
   <212> PRT
   <213> Homo Sapiens
<400> 18
   Leu Ile Ser Glu Val Asp Ser Asp Gly Asp Gly Glu
   1 5 10
<210> 19
   <211> 23
   <212> PRT
   <213> Homo Sapiens
<400> 19
   Leu Ile Ser Glu Val Asp Ser Asp Gly Asp Gly Glu Ile Ser Phe Gln
   1 5 10 15
   Glu Phe Leu Thr Ala Ala Lys
   20
<210> 20
   <211> 23
   <212> PRT
   <213> Homo Sapiens
<400> 20
   Leu Ile Ser Glu Val Asp Ser Asp Gly Asp Gly Glu Ile Ser Phe Gln
   1 5 10 15
   Glu Phe Leu Thr Ala Ala Arg
   20
<210> 21
   <211> 8
   <212> PRT
   <213> Homo Sapiens
<400> 21
   Asn Leu Ser Glu Ala Gln Leu Arg
   1 5
<210> 22
   <211> 8
   <212> PRT
   <213> Homo Sapiens
<400> 22
   Asn Leu Ser Glu Ala Gln Leu Arg
   1 5
<210> 23
   <211> 12
   <212> PRT
   <213> Homo Sapiens
<400> 23
   Ser Ala Val Asp Thr Asp Gly Asn Gly Thr Ile Asn
   1 5 10
<210> 24
   <211> 21
   <212> PRT
   <213> Homo Sapiens
<400> 24
   Ser Ala Val Asp Thr Asp Gly Asn Gly Thr Ile Asn Ala Gln Glu Leu
   1               5                   10                  15
   Gly Ala Ala Leu Lys
               20
<210> 25
   <211> 8
   <212> PRT
   <213> Homo Sapiens
<400> 25
   Val Asn Tyr Glu Glu Phe Ala Arg
   1               5